# EUROPEAN PATENT APPLICATION

(11) **EP 1 693 106 A1**
(43) Date of publication of application: **23.08.2006**
(21) Application number: 06250480.8
(22) Date of filing: 28.01.2006
(51) Int. Cl.: B01J 37/34, B01J 23/16, C07C 51/215, C07C 51/25

(54) **Process for preparing catalysts for the partial oxidation of alkanes and alkenes**

(30) Priority: 11.02.2005 US 652160 P
(71) Applicant: ROHM AND HAAS COMPANY, Philadelphia, Pennsylvania 19106-2399 (US)
(72) Inventor: Cavalcanti, Fernando Antonio Pessoa, Lafayette Hill Pennsylvania 19444-2047 (US); Devlin, A. Marie, Hatfield, Pennsylvania 19440 (US); Heffner, Michele Doreen, Chalfont, Pennsylvania 18914 (US)
(74) Representative: Kent, Venetia Katherine

(57) **Abstract**

A process for preparing mixed metal oxide catalysts is described that combines microwave irradiation and hydrothermal processing of one or more mixed metal oxide precursors.

## Description

The present invention relates to a process for preparation of mixed-metal oxides useful as catalysts for partial oxidation of alkanes, alkenes and mixtures thereof. In particular, the process of the present invention involves irradiating catalyst precursors with microwave energy prior to or simultaneously with calcination. The present invention also relates to mixed metal oxide catalysts produced by the aforesaid process, which have improved performance characteristics, including improved efficiency and selectivity for converting alkanes, alkenes and mixtures thereof to their corresponding oxygenates.

The selective partial oxidation of alkenes to unsaturated carboxylic acids and their corresponding esters is an important commercial process. However, the selective and efficient partial oxidation/dehydrogenation of alkanes to products including olefins, unsaturated carboxylic acids and esters of unsaturated carboxylic acids is not optimized in large part due to an optimal catalyst and has remained a catalytic process with a number of challenges to overcome.

International Publication No. 99/00326 discloses initiating a redox reaction between a plurality of metal salts in aqueous solution which requires at least one strong oxidizing agent and at least one strong reducing agent using microwave energy, wherein the metal oxide product is produced by establishing the redox couple. This publication, however, fails to disclose any methods for controlling the particle size of the material being irradiated with microwave energy. Furthermore, this publication fails to disclose or suggest the use of microwave energy for successful synthesis of mixed metal oxide catalysts which are suitable for partial oxidation of alkanes, alkenes, and mixtures thereof.

In addition, heat transfer in conventional hydrothermal synthesis of mixed metal oxides is often not optimal or uniform and such methods of preparation require long periods of time for heating precursors and corresponding long periods of time for cooling the mixed metal oxides formed hydrothermally. It is desirable, therefore, to provide new methods for providing rapid and uniform heat transfer in hydrothermal methods using microwave irradiation.

Accordingly, the present invention provides a process for preparing a mixed metal oxide catalysts, comprising the steps of:
a) providing a solution comprising one or more metal oxide catalyst precursors and a solvent;
b) adjusting the pH of the solution to between 1.0 and 4.0; ???
c) heating the solution by irradiating the solution with microwave energy having one or more microwave frequencies; and
d) removing the solvent from the solution.

The mixed metal oxide catalysts prepared using the method of the present invention are useful for catalytically converting alkanes, alkenes, and combinations of alkanes and alkenes, to their corresponding oxygenates.

According to one embodiment of the invention, catalysts prepared in accordance with the process of the present invention are one or more mixed metal oxide catalysts having a catalyst having the empirical formula

MoVₐNb_{b}X_{c}Z_{d}Oₙ

wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements.

Known, conventional methods for preparation of mixed metal oxide (MMO) catalysts, as well as the compositions of such MMO catalysts, are described in, for example, U. S. Patent Nos. 6,383,978; 6,461,996; 6,518,216; 6,403,525; 6,407,031; 6,407,280; 6,589,907; 6,746,983; and 6,818,588. Such known methods typically involve the steps of forming a precursor slurry, by any one of various known methods including, but not limited to, hydrothermal synthesis, co-precipitation, and sol-gel processing; removing the solvent (often, but not necessarily, water) from the slurry (this step is also referred to as drying the precursor slurry), to obtain a dried precursor; and, finally, calcining the dried precursor to obtain an MMO catalyst.

The initial step of forming a precursor slurry typically involves the component steps of providing a solution comprising one or more metal oxide catalyst precursors and a solvent; and then heating that solution for some period of time. As is understood by persons of ordinary skill in the art, hydrothermal synthesis is a catalyst synthesis method which involves heating the mixed precursor solutions in a closed vessel under pressure. However, the solution may, alternatively, be heated in a vessel that is open and under atmospheric, or sub-atmospheric, pressure. In either case, the heating may be conducted using any known, conventional means, such as, resistance heaters, coil heaters, fuel fired heaters, etc.

In the present invention, the heating step is accomplished by irradiating the solution with microwave energy having one or more microwave frequencies. The step of heating by microwave irradiation may occur within a microwave chamber.

For the irradiating step, the frequency, dimensions, and power level (constant, oscillating, ramping, instantaneously maximizing, etc.) of the microwave energy during the heating by irradiation step, may be varied to optimize the catalyst's physical characteristics, composition, crystallinity, and the like. Both inorganic and organic templates may be used to control and direct the morphology of the catalyst precursor and final catalyst.

A precursor slurry of mixed metal salts is first prepared by conventional methods and methods described above that include, but are not limited to, hydrothermal methods, co-precipitation, sol gel processing and combinations thereof. After the precursor slurry is prepared it is dried according to conventional drying methods including, but not limited to, drying in ovens, spray drying, freeze drying, and rotary evaporation. The dried precursor is then calcined to obtain prepared MMO catalysts using well-known techniques and techniques described above to those having skill in the art including, but not limited to for example, flow calcinations, static calcinations, rotary calcinations and fluid-bed calcinations. In some cases the prepared MMO catalysts are further milled to improve their catalytic activity.

In particular, the invention is directed to a method for preparing mixed metal oxide catalysts by applying microwave radiation during hydrothermal synthesis of the mixed metal oxide catalysts from catalyst precursors. By irradiating mixed metal oxide catalyst precursors at microwave frequencies during hydrothermal synthesis, mixed metal oxide catalysts are produced having improved reactivities and selectivities as compared to metal oxide catalysts prepared using conventional hydrothermal methods.

This new synthesis approach is useful for the optimization of known mixed metal oxide compositions (MMOs), such as, without limitation, Mo-V-Te-Nb-based mixed metal oxide catalysts, Mo-V-Sb-Nb-based mixed metal oxide catalysts, as well as three component MMOs (e.g., Mo-V-Te-Ox and Mo-V-Nb-Ox). It is also useful for the synthesis of new MMOs that may serve as selective oxidation catalysts (e.g., the conversion of propane to acrylic acid).

Once the resulting mixed metal oxide catalysts are formed, liquid therein is removed by any suitable method, known in the art, for forming a catalyst precursor. Such methods include, without limitation, vacuum drying, freeze drying, spray drying, rotary evaporation and air-drying. Vacuum drying is generally performed at pressures ranging from 10 mm Hg to 500 mm Hg. Freeze drying typically entails freezing the slurry or solution, using , for instance, liquid nitrogen, and drying the frozen slurry or solution under vacuum. Spray drying is generally performed under an inert atmosphere such as nitrogen or argon, with an inlet temperature ranging from 125°C to 200°C and an outlet temperature ranging from 75°C to 150°C. Rotary evaporation is generally performed at a bath temperature of from 25°C to 90°C and at a pressure of from 10 mm Hg to 760 mm Hg, preferably at a bath temperature of from 40° to 90°C and at a pressure of from 10 mm Hg to 350 mm Hg, more preferably at a bath temperature of from 40°C to 60°C and at a pressure of from 10 mm Hg to 40 mm Hg. Air drying may be effected at temperatures ranging from 25°C to 90°C. Rotary evaporation or air-drying are generally preferred.

Once obtained, the resulting mixed metal oxide catalyst precursor may be used, or it may be further processed by conventional techniques which are well-known to persons of ordinary skill in the relevant art, including further milling and calcining.

According to one embodiment, calcination may be conducted in an oxygen-containing atmosphere or in the substantial absence of oxygen, e.g., in an inert atmosphere or in vacuo. The inert atmosphere may be any material which is substantially inert, i.e., does not react or interact with, the catalyst precursor. Suitable examples include, without limitation, nitrogen, argon, xenon, helium or mixtures thereof. Preferably, the inert atmosphere is argon or nitrogen. The inert atmosphere may flow over the surface of the catalyst or may not flow thereover (a static environment). When the inert atmosphere does flow over the surface of the catalyst precursor, the flow rate can vary over a wide range, e.g., at a space velocity of from 1 to 500 hr⁻¹.

Calcination of mixed metal oxide catalysts is usually performed at a temperature of from 350°C to 850°C, preferably from 400°C to 700°C, more preferably from 500°C to 640°C. The calcination is performed for an amount of time suitable to form the aforementioned catalyst. Typically, the calcination is performed for from 0.5 to 30 hours, preferably from 1 to 25 hours, more preferably for from 1 to 15 hours, to obtain the desired promoted mixed metal oxide.

According to one embodiment, the MMO catalyst may be calcined in two stages. In the first stage, the catalyst precursor is calcined in an oxidizing environment (e.g. air) at a temperature of from 200°C to 400°C, preferably from 275°C to 325°C for from 15 minutes to 8 hours, preferably for from 1 to 3 hours. In the second stage, the material from the first stage is calcined in a non-oxidizing environment (e.g., an inert atmosphere) at a temperature of from 500°C to 700°C, preferably for from 550°C to 650°C, for 15 minutes to 8 hours, preferably for from 1 to 3 hours. Optionally, a reducing gas, such as, for example, ammonia or hydrogen, may be added during the second stage calcination.

According to a separate embodiment, a suitable mixed metal oxide catalyst may be obtained through cryo-grinding (also referred to a freeze milling). There is no particular restriction as to the grinding method, and conventional methods may be employed. As a dry grinding method, a method of using a gas stream grinder may, for example, be mentioned wherein coarse particles are permitted to collide with one another in a high speed gas stream for grinding. The grinding may be conducted not only mechanically but also by using a mortar or the like in the case of a small scale operation.

As a wet grinding method wherein grinding is conducted in a wet state by adding water or an organic solvent to the above mixed metal oxide, a conventional method of using a rotary cylinder-type medium mill or a medium-stirring type mill, may be mentioned. The rotary cylinder-type medium mill is a wet mill of the type wherein a container for the object to be ground is rotated, and it includes, for example, a ball mill and a rod mill. The medium-stirring type mill is a wet mill of the type wherein the object to be ground, contained in a container is stirred by a stirring apparatus, and it includes, for example, a rotary screw type mill, and a rotary disc type mill.

The conditions for grinding may suitably be set to meet the nature of the above-mentioned promoted mixed metal oxide, the viscosity, the concentration, etc. of the solvent used in the case of wet grinding, or the optimum conditions of the grinding apparatus. However, it is preferred that grinding is conducted until the average particle size of the ground catalyst precursor would usually be at most 20µm, more preferably at most 5µm. Improvement in the catalytic performance occurs due to such cryo-grinding.

Further, in some cases, it is possible to further improve catalytic activities by further adding a solvent to the ground catalyst precursor to form a solution or slurry, followed by drying again. There is no particular restriction as to the concentration of the solution or slurry, and it is usual to adjust the solution or slurry so that the total amount of the starting material compounds for the ground catalyst precursor is from 10 to 60 wt. %. Then, this solution or slurry is dried by a method such as spray drying, freeze drying, evaporation to dryness or vacuum drying, preferably by the spray drying method. Further, similar drying may be conducted also in the case where wet grinding is conducted.

The mixed metal oxide (promoted or not) catalyst obtained by the above-mentioned method may be used as a final catalyst, but it may further be subjected to one or more additional chemical, physical and combinations of chemical and physical treatments. According to one embodiment, the catalysts are further modified using heat treatment. As an exemplary embodiment, heat treatment usually is performed at a temperature of from 200° to 700°C for from 0.1 to 10 hours.

The resulting modified mixed metal oxide (promoted or not) may be used by itself as a solid catalyst. The modified catalysts may also be combined with one or more suitable carriers, such as, without limitation, silica, alumina, titania, aluminosilicate, diatomaceous earth or zirconia, according to art-disclosed techniques. Further, it may be processed to a suitable shape or particle size using art disclosed techniques, depending upon the scale or system of the reactor.

Alternatively, the metal components of the MMO catalysts prepared in accordance with the process of the present invention may be supported on materials such as alumina, silica, silica-alumina, zirconia, titania, etc., for example, by conventional incipient wetness techniques. In one typical method, solutions containing the metals are contacted with the dry support such that the support is wetted; then, the resultant wetted material is dried, for example, at a temperature from room temperature to 200°C followed by calcination as described above. In another method, metal solutions are contacted with the support, typically in volume ratios of greater than 3 : 1 (metal solution : support), and the solution agitated such that the metal ions are ion-exchanged onto the support. The metal-containing support is then dried and calcined as detailed above.

The prepared metal oxide catalysts are modified using the one or more chemical, physical and combined chemical and physical treatments to provide modified mixed metal oxide catalysts

According to a separate embodiment, MMO catalysts may also be prepared using one or more promoters. The starting materials for the above promoted mixed metal oxide are not limited to those described above. A wide range of materials including, for example, oxides, nitrates, halides or oxyhalides, alkoxides, acetylacetonates, and organometallic compounds may be used. For example, ammonium heptamolybdate may be utilized for the source of molybdenum in the catalyst. However, compounds such as MoO₃, MoO₂, MoCl₅, MoOCl₄, Mo(OC₂H₅)₅, molybdenum acetylacetonate, phosphomolybdic acid and silicomolybdic acid may also be utilized instead of ammonium heptamolybdate. Similarly, ammonium metavanadate may be utilized for the source of vanadium in the catalyst. However, compounds such as V₂O₅, V₂O₃, VOCl₃, VCl₄, VO(OC₂H₅)₃, vanadium acetylacetonate and vanadyl acetylacetonate may also be utilized instead of ammonium metavanadate. The tellurium source may include telluric acid, TeCl₄, Te(OC₂H₅)₅, Te(OCH(CH₃)₂)₄ and TeO₂. The niobium source may include ammonium niobium oxalate, Nb₂O₅, NbCl₅, niobic acid or Nb(OC₂H₅)₅ as well as the more conventional niobium oxalate.

In addition, with reference to the promoter elements for the promoted catalyst, the nickel source may include nickel(II) acetate tetrahydrate, Ni(NO₃)₂, nickel(II) oxalate, NiO, Ni(OH)₂, NiCl₂, NiBr₂, nickel(II) acetylacetonate, nickel(II) sulfate, NiS or nickel metal. The palladium source may include Pd(NO₃)₂, palladium(II) acetate, palladium oxalate, PdO, Pd(OH)₂, PdCl₂, palladium acetylacetonate or palladium metal. The copper source may be copper acetate, copper acetate monohydrate, copper acetate hydrate, copper acetylacetonate, copper bromide, copper carbonate, copper chloride, copper chloride dihydrate, copper fluoride, copper formate hydrate, copper gluconate, copper hydroxide, copper iodide, copper methoxide, copper nitrate, copper nitrate hydrate, copper oxide, copper tartrate hydrate or a solution of copper in an aqueous inorganic acid, e.g., nitric acid. The silver source may be silver acetate, silver acetylacetonate, silver benzoate, silver bromide, silver carbonate, silver chloride, silver citrate hydrate, silver fluoride, silver iodide, silver lactate, silver nitrate, silver nitrite, silver oxide, silver phosphate or a solution of silver in an aqueous inorganic acid, e.g., nitric acid. The gold source may be ammonium tetrachloroaurate, gold bromide, gold chloride, gold cyanide, gold hydroxide, gold iodide, gold oxide, gold trichloride acid and gold sulfide.

Catalysts prepared in accordance with the process of the present invention have different chemical, physical and performance characteristics in catalytic reactions of carbon based molecules as compared to known catalysts. According to one embodiment, the treated catalyst exhibits changes in X-ray lines, peak positions and intensity of such lines and peaks as compared with corresponding X-ray diffraction data for corresponding known catalysts. Such difference indicate structural differences between the catalyst prepared by the processes of the present invention and known catalysts and are evidenced by the catalytic activity and selectivity. For example, compared with an untreated catalyst composition, a treated catalyst composition of the present invention exhibits an X-ray diffraction pattern having a relative increase in a diffraction peak at a diffraction angle (2θ) of 27.1 degrees when compared with an untreated catalyst, which may exhibit no peak at all at 27.1 degrees.

The relative difference between peak intensities of treated versus untreated compositions may be greater than 5%, more preferably greater than 10%, and still more preferably greater than 20% of the intensity of the untreated catalyst composition at the diffraction angle (2θ) of 27.1 degrees. Without intending to be bound by theory, it is believed that at least two phases (A and B) are present in the resulting mixed metal oxide catalyst and the treatment of the catalyst precursor with a source of NOₓ results in an increase in phase B relative to phase A in the resulting catalyst. The increase in phase B is believed to contribute to improved performance of the catalyst in terms of selectivity, reactivity and yield.

Catalysts prepared in accordance with the process of the present invention exhibit improved catalyst performance characteristics selected from the group consisting of: optimized catalyst properties, yields of oxygenates including unsaturated carboxylic acids, from their corresponding alkanes, alkenes or combinations of corresponding alkanes and alkenes at constant alkane/alkene conversion, selectivity of oxygenate products, including unsaturated carboxylic acids, from their corresponding alkanes, alkenes or combinations of corresponding alkanes and alkenes, optimized feed conversion, cumulative yield of the desired oxidation product, optimized reactant/product recycle conversion, optimized product conversion via recycle and combinations thereof, as compared to the known catalyst.

The following illustrative examples are provided to further demonstrate the utility of the present invention and are not in any way construed to be limiting. Moreover, the examples provided are representative examples that broadly enable the claimed scope of the invention. In the following Examples, "propane conversion" is synonymous with "feed conversion" and was calculated in accordance with the formulas provided earlier hereinabove. Furthermore, "AA yield" means acrylic acid yield and is synonymous with "product yield" and was calculated in accordance with the formulas provided earlier hereinabove.

Unless otherwise specified, all percentages recited in the following Examples are by volume, based on the total volume of the feed or product gas stream.

### EXAMPLES

### Catalyst Preparation:

Ammonium heptamolybdate, ammonium vanadate (or, alternatively, vanadyl sulfate) and telluric acid (or, alternatively, tellurium oxide) are added to deionized water. The mixture is heated to 70°C, to ensure the dissolution of all starting materials, and then cooled back to 30°C. Ammonium niobium oxalate salt is added directly to this solution. The use of additional oxalic acid in not necessary but it may be used if so desired. The pH of this mixture is then adjusted to the desired value, usually between 1.0 and 3.5, but most preferably between 2.0 and 2.5, using an acid such as HNO₃, HCl, H₂SO₄, H₃PO₄, HClO₄, etc. This solution is then charged directly to the microwave or convection hydrothermal vessels which are subjected to a short heating program, such as heating to 175°C over 30 minutes and maintaining this temperature for 90 minutes. The vessels are then allowed to cool down to room temperature and their solid content is recovered by filtration. This precursor solid is then handled in the same manner as similar precursors produced by techniques such as spray drying and Rotavap to produce the final catalyst.

The catalysts for the following examples had nominal compositions, based on the elements charged in the starting materials, of MoV_{0.25}Te_{0.17}Nb_{0.15}Oₓ. All catalysts were calcined, ground (cryogrinding) and extracted (methanol/oxalic acid) in the exact same manner. They are designated and differentiated as follows:
- Cat A: no pH adjustment - microwave hydrothermal synthesis.
- Cat B: no pH adjustment - convection hydrothermal synthesis.
- **Cat C**: pH adjustment to 2.1 with HNO₃ - microwave hydrothermal synthesis.
- Cat D: pH adjustment to 2.1 with HNO₃ - convection hydrothermal synthesis.
- Cat E: pH adjustment to 2.1 with HCl - microwave hydrothermal synthesis.
- Cat F: no pH adjustment - vanadyl sulfate used instead of ammonium vanadate - microwave hydrothermal synthesis.
- **Cat G**: pH adjustment to 2.1 with HNO₃ - tellurium oxide used instead of telluric acid - microwave hydrothermal synthesis.

Table 2 below summarizes the results of the evaluation of these catalysts in a 7% propane SDF system.

**Table 2.**

| | **Reactor Temperature, °C** | **Propane Conversion, %** | **AA Selectivity, %** | **AA Yield, %** |
|---|---|---|---|---|
| **Cat A** | 384 | 52.3 | 72.7 | 38.0 |
| | 394 | 61.3 | 67.7 | 41.5 |
| | 397 | 64.1 | 65.3 | 41.8 |
| **Cat B** | 382 | 47.6 | 70.7 | 33.6 |
| | 390 | 54.2 | 67.1 | 36.3 |
| | 395 | 58.3 | 63.7 | 37.1 |
| **Cat C** | 354 | 53.8 | 74.9 | 40.3 |
| | 362 | 62.0 | 71.4 | 44.3 |
| | 368 | 68.5 | 67.0 | 45.9 |
| **Cat D** | 346 | 50.4 | 73.8 | 37.2 |
| | 356 | 61.7 | 69.8 | 43.1 |
| | 361 | 68.4 | 65.5 | 44.8 |
| **Cat E** | 338 | 50.4 | 67.9 | 34.2 |
| | 348 | 61.5 | 63.6 | 39.1 |
| | 353 | 68.1 | 60.1 | 40.9 |
| **Cat F** | 384 | 48.2 | 63.1 | 30.4 |
| | 392 | 56.2 | 56.1 | 31.5 |
| | 397 | 61.1 | 50.4 | 30.8 |
| **Cat G** | 348 | 53.7 | 74.1 | 39.8 |
| | 353 | 59.5 | 72.3 | 43.0 |
| | 361 | 69.6 | 66.4 | 46.2 |

It will be understood that the embodiments of the present invention described hereinabove are merely exemplary and that a person skilled in the art may make variations and modifications without departing from the spirit and scope of the invention. All such variations and modifications are intended to be included within the scope of the present invention.

## Claims

1. A process for preparing a mixed metal oxide catalyst, comprising the steps of:
a) providing an aqueous solution of one or more metal oxide catalyst precursors;
b) adjusting the pH of the solution of one or more metal oxide catalyst precursors to between 1.0 and 4.0;
c) irradiating the aqueous solution of one or more metal oxide catalyst precursors in a microwave chamber at one or more microwave frequencies; and
d) recovering the mixed metal oxide catalyst.

2. The process according to claim 1, further comprising the additional step of calcining the mixed metal oxide catalyst.

3. The process according to claim 1, wherein the mixed metal oxide catalysts comprises the empirical formula:
MₑMoVₐNb_{b}X_{c}Z_{d}Oₙ
wherein Mₑ is at least one or more chemical modifying agents, X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, 0.1 ≤ a ≤ 1.0, 0.01 ≤ b ≤ 1.0, 0.01 ≤ c ≤ 1.0, 0 ≤ d ≤ 1.0 and n, e are determined by the oxidation states of the other elements.

4. The process according to claim 1, wherein the mixed metal oxide catalysts comprises the empirical formula: MoV_{0.25}Te_{0.17}Nb_{0.15}Oₓ.

5. The process according to claim 1, wherein the pH of the one or more catalyst precursors is adjusted to between 2.0 to 2.5.

6. The process according to claim 1, wherein ammonium vanadate and tellurium oxide are used as mixed metal oxide precursors.
